# EUROPEAN PATENT SPECIFICATION

(11) **EP 2 539 443 B1**
(45) Date of publication and mention of the grant of the patent: **27.03.2019**
(21) Application number: 11746961.9
(22) Date of filing: 23.02.2011
(51) Int. Cl.: A61K 38/18, A61K 35/35, A61K 8/64, A61K 9/00, A61Q 19/08, A61P 17/02, A61P 41/00

(54) **ERYTHROPOIETIN FOR USE IN ENHANCING FAT GRAFT SURVIVAL**
ERYTHROPOIETIN ZUR VEWENDUNG ZUR FÖRDERUNG VON FETTTRANSPLANT-ÜBERLEBENS
ERYHTROPOIETINE POUR AMÉLIORER LA SURVIE D'UN GREFFON ADIPEUX

(30) Priority: 23.02.2010 US 306991 P
(43) Date of publication of application: 02.01.2013
(73) Proprietor: SEBANA MEDICAL LTD., 17111 Nazareth IIit (IL)
(72) Inventor: HAMED, Saher, 17091 Nazareth Ilit (IL)
(74) Representative: Patentanwälte Bressel und Partner mbB
(86) International application number: PCT/IL2011/000181
(87) International publication number: WO 2011/104707

(56) References cited:
- WO-A2-2005/011569
- WO-A2-2009/022338
- US-A1- 2005 048 034
- US-A1- 2009 018 033
- US-B2- 7 459 152
- HAMED SAHER ET AL: "Erythropoietin improves the survival of fat tissue after its transplantation in nude mice.", PLOS ONE 2010, vol. 5, no. 11, E13986, November 2010 (2010-11), pages 1-12, XP002715355, ISSN: 1932-6203

## Description

### FIELD AND BACKGROUND OF THE INVENTION

The present invention relates to Erythropoietin for use in enhancing implanted fat cell survival for reconstruction surgery in a subject in need thereof.

During angiogenesis, endothelial cells change their phenotype to an angiogenic phenotype that includes the production of proteases, such as matrix metalloproteinases (MMPs), and the ability to migrate and proliferate. This process is dependent upon the activity of several growth factors, such as vascular endothelial growth factor (VEGF), basic fibroblast growth factor (bFGF) and platelet-derived growth factor (PDGF)-BB.

Erythropoietin (EPO), a glycoprotein hormone that stimulates erythropoiesis, has been reported to possess angiogenic activity. Ribatti and colleagues demonstrated that EPO can induce a pro-angiogenic phenotype in cultured endothelial cells and stimulate angiogenesis in vivo [Ribatti et al. (2003) Eur J Clin Invest 33:891-896]. EPO has also been shown to indirectly stimulate angiogenesis in ischemic tissue by increasing the expression of VEGF protein and recruiting endothelial progenitor cells [Nakano et al. (2007) Circ Res 100:662-669; Aicher et al. (2005) Hypertension 45:321- 325]. In rats, EPO administration has also been shown to mobilize bone marrow- derived progenitor cells [Hamed et al. (2006) Eur Heart J 27:1876-83] and to increase the myocardial expression of VEGF [Westenbrink et al. (2007) Eur Heart J 28:2018- 2027]. Wang and colleagues demonstrated that EPO can promote angiogenesis by stimulating VEGF secretion from neural progenitor cells and VEGF-receptor expression in cerebral endothelial cells [Wang et al. (2008) J Cereb Blood Flow Metab 28:1361-8]. Collectively, these results suggest that EPO is an indirectly-acting angiogenic factor whose actions are mediated by stimulating the secretion of angiogenic factors.

EPO has also been reported to possess other non-hematopoietic effects, including cytoprotection of vascular endothelial cells [Chong et al. (2003) Curr Drug Targets Cardiovasc Haematol Disord 3:141-154] and an anti-apoptotic action in vascular smooth muscle cells and in endothelial cells [Somervaille et al. (2001) Blood 98:1374-1381]. These anti-apoptotic actions include prevention of mitochondrial release of cytochrome c, suppression of caspase activity, upregulation of protein kinase B (PKB) signaling pathway activity and the expression of the antiapoptotic protein Bcl-xl.

Autologous fat transplantation is a common and ideal technique for soft tissue augmentation and for filling soft tissue defects due to trauma or aging. Emerging evidence suggests that early and adequate vascularization of the fat graft is essential for its take and viability. However, the relatively high resorption rate of the fat graft, due to increased fat cell death after transplantation, reduces the efficacy of this technique [Nishimura et al. (2000) Laryngoscope 110:1333-1338]. Although angiogenic factors [Rophael et al. (2007) Am J Pathol 171:2048-2057; Kuramochi et al. (2008) Eur J Clin Invest 38:752-759], as well as VEGF gene therapy [Lei et al. (2008) Chin J Traumatol 11:49-53; Lu et al. (2009) Plast Reconstr Surg 124:1437-1446; Yi et al. (2007) J Plast Reconstr Aesthet Surg 60:272-278] have been individually used to stimulate angiogenesis in fat grafts in order to enhance fat cell survival and viability, the clinical outcome has been disappointing [Henry et al. (2003) Circulation 107:1359-1365]. Therefore, reducing the resorption rate of transplanted fat is a clinical challenge.

Various approaches of improving grafting have been attempted, some are summarized infra.

PCT Publication No. 2005/018549 discloses methods and compositions for tissue repair (e.g. bone, cartilage). According to their teachings, a tissue graft (e.g. fat tissue, muscle tissue) is contacted ex vivo with one or more bioactive agents (e.g. erythropoietin) thereby stimulating at least a portion of the cells in the tissue to differentiate into cells of a desired type (e.g. bone cells) and then the tissue is implanted into a subject.

U.S. Pat. No. 7459152 discloses erythropoietin administration for improved graft survival. According to their teachings, cells of a tissue graft (e.g. cells of a neural or paraneural origin, such as adrenal chromaffin cells) are treated with erythropoietin before, during or after delivery or administration into a subject for the treatment of neurological diseases (e.g. Parkinson's disease, Alzheimer's disease, spinal cord injury).

U.S. Pat. No. 5,681,561 discloses method and compositions for improving autologous fat grafting. According to the teachings of U.S. Pat. No. 5,681,561, autologous fat cells (e.g. lipocytes) are injected into a patient along with a non-steroidal anabolic hormone (e.g. insulin or triiodothyronine/thyroxine or both). The autologous fat cells may further be injected into a subject with a growth hormone [e.g. epithelial growth factor (EGF), platelet derived growth factor (PDGF)]. In addition, the hormones are combined with a nutrient medium.

PCT Publication No. 2008/019434 discloses use of agents to enhance adipogenesis and to promote fat graft survival. According to their teachings, growth factors [e.g. platelet-derived growth factor (PDGF), vascular endothelial growth factor (VEGF) and/or fibroblast growth factor (FGF)] are delivered by local or sustained administration to enhance angiogenesis in association with adipogenesis and to promote fat graft survival.

In US 2005/048034 A1 cells present in adipose tissue are used to promote wound healing in a patient. Methods of treating patients include processing adipose tissue to deliver a concentrated amount of regenerative cells obtained from the adipose tissue to a patient. The methods may be practiced in a closed system so that the regenerative cells are not exposed to an external environment prior to being administered to a patient. Accordingly, in one method, cells present in adipose tissue are placed directly into a recipient along with such additives necessary to promote, engender or support a therapeutic benefit.

WO 2005/011569 A2 discloses methods of treating patients for conditions such as breast augmentation, soft tissue defects, and urinary incontinence. The methods include removing adipose tissue from a patient, processing a portion of the adipose tissue to obtain a substantially isolated population of regenerative cells, mixing the regenerative cells with another portion of adipose tissue to form a composition, and administering the composition to the patient from which the adipose tissue was removed.

### SUMMARY OF THE INVENTION

The present invention is directed to Erythropoietin for use in enhancing implanted fat cell survival for reconstruction surgery in a subject in need thereof, wherein a dose of Erythropoietin for local administration ranges between 1-1000 IU per injection per 1,000,000 fat cells or wherein the dose of Erythropoietin for systemic administration ranges between 10 - 7500 IU per kg body weight.

The present invention is also directed to the use of Erythropoietin in enhancing implanted fat cell survival for treating wrinkles in a subject in need thereof, wherein a dose of Erythropoietin for local administration ranges between 1-1000 IU per injection per 1,000,000 fat cells or wherein the dose of Erythropoietin for systemic administration ranges between 10 - 7500 IU per kg body weight, wherein the use is non-therapeutic.

According to the present invention there is provided a pharmaceutical composition comprising a population of fat cells and Erythropoietin at a dose of about 1-1000 IU per injection per 1,000,000 fat cells.

According to some embodiments of the use of the invention, fat cells are treated with Erythropoietin prior to the implanting.

According to the invention, Erythropoietin may be directly injected into the population of fat cells.

According to the invention, administering may be effected at least twice.

According to some embodiments of the invention, the use further comprises administering to the subject at least one factor selected from the group consisting of an extracellular matrix component, a growth factor, a hormone, an angiogenic factor, a coagulation factor, a cytokine, a chemokine, an enzyme, a neurotransmitter, a vitamin, a carbohydrate, an ion, an iron chelator, a fatty acid, an antibiotic and an amino acid.

According to the invention, the soft tissue defect may be selected from the group consisting of a skin condition, a skin malady, a wound, a burn, a cancer, a surgery, a reconstruction surgery, a skin depression, a congenital malformation and an acquired disease.

According to some embodiments of the invention, the fat cell comprises an autologous cell.

According to some embodiments of the invention, the fat cell comprises a non- autologous cell.

According to some embodiments of the invention, the non-autologous cell is an allogeneic cell.

According to some embodiments of the invention, the non-autologous cell is a xenogeneic cell.

According to the invention, the non-autologous cell may be obtained from a mammal.

According to some embodiments of the invention, the pharmaceutical composition comprises at least one factor selected from the group consisting of an extracellular matrix component, a growth factor, a hormone, an angiogenic factor, a coagulation factor, a cytokine, a chemokine, an enzyme, a neurotransmitter, a vitamin, a carbohydrate, an ion, an iron chelator, a fatty acid, an antibiotic, and an amino acid.

Unless otherwise defined, all technical and/or scientific terms used herein have the same meaning as commonly understood by one of ordinary skill in the art to which the invention pertains. Although uses and materials similar or equivalent to those described herein can be applied in the practice or testing of embodiments of the invention, exemplary uses and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

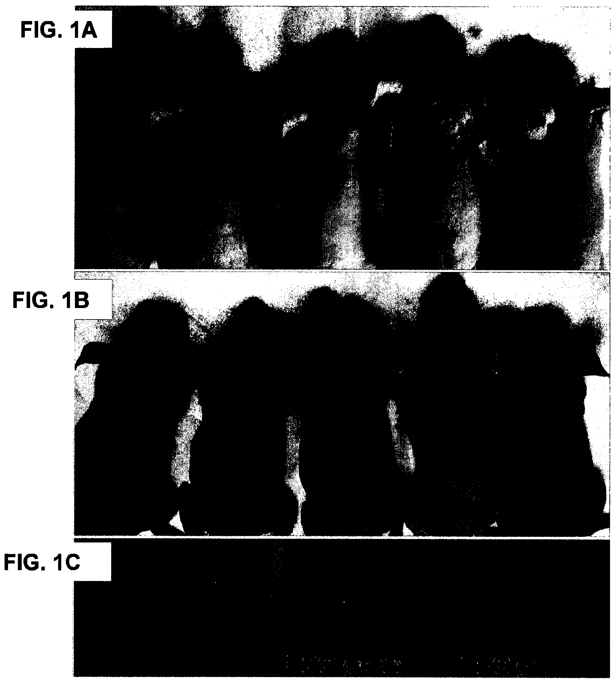

FIGs. 1A-C are photographs depicting five representative mice with fat grafts at the end of the 15-week study period. Figure 1A shows five PBS-treated fat grafts with small lumps that vary in their size in the scalps. Figure 1B shows five high-dose erythropoietin (100 IU EPO)-treated fat grafts with large lumps that are similar in their size in the scalps. Figure 1C shows fat grafts which were dissected from the mice 15 weeks after transplantation. From left to right: a representative small fat graft from a PBS-treated fat graft, an intermediate-size low-dose EPO-treated fat graft, and a large high-dose EPO-treated fat graft respectively. Scale bar: 10 mm.

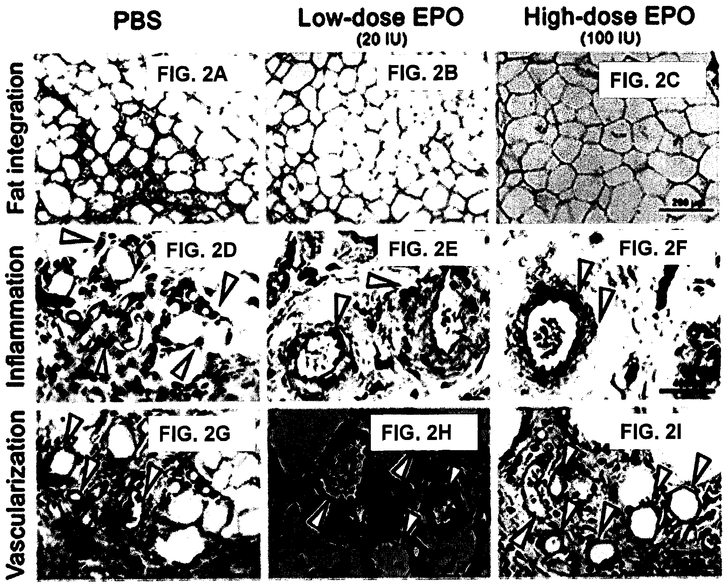

FIGs. 2A-C are photographs depicting histological sections of fat grafts that were removed from the PBS-treated, low-dose EPO treated and high-dose EPO treated mice 15 week after fat transplantation. Sections were stained with hematoxylin and eosin, and were examined under light microscope for: (i) the extent of integration, as evidenced by the extent of organization of intact and nucleated fat cells in the grafted fat tissue architecture; (ii) the extent of fibrosis, as evidenced by the amount of collagen and elastic fibrils; (iii) the presence of cysts and vacuoles; and (iiii) the intensity of the inflammatory response, as evidenced by the extent of lymphocyte and macrophage infiltration. Each criterion was graded on a scale of 0 to 5 where 0 = absence, 1 = minimal presence, 2 = minimal to moderate presence, 3 = moderate presence, 4 = moderate to extensive, and 5 = extensive presence. Representative histological micrograph are shown as follows: Figure 2A, a PBS-treated fat graft in which there is fat cell degeneration, fibrosis, and infiltration of nucleated inflammatory cells although some cells are still viable and intact; Figure 2B, a low dose erythropoietin (EPO)-treated fat graft in which the fat cells are well-defined in tissue in which there is a moderate amount of fibrosis; and Figure 2C, a high dose EPO-treated fat graft in which there are viable, well-defined intact fat cells with modest amounts of connective tissue. Scale bar: 200µm.

FIGs. 2D-F are photographs depicting the effect of erythropoietin (EPO) on inflammatory response in fat grafts after fat transplantation. Following implantation into three groups of mice, fat grafts were treated with either PBS (100 µl, Figure 2D), 20 IU EPO/100 µl PBS (low-dose, Figure 2E), or 100 IU EPO/100 µl PBS (high-dose, Figure 2F) on the day of fat injection and repeatedly every three days for a total of 18 days. After harvesting the fat grafts, sections were prepared for assessing inflammatory response as evidenced by CD68-positive cell infiltration. The arrows are pointing to brown-stained CD68-positive cells.

FIGs. 2G-I are photographs depicting the effect of erythropoietin (EPO) on new blood vessel formation in fat grafts after fat transplantation. Following implantation into three groups of mice, fat grafts were treated with either PBS (100 µl, Figure 2G), 20 IU EPO/100 µl PBS (low-dose, Figure 2H), or 100 IU EPO/100 µl PBS (high-dose, Figure 2I) on the day of fat injection and repeatedly every three days for a total of 18 days. After harvesting the fat grafts, sections were prepared for assessing microvascular density (MVD). The arrows are pointing to brown-stained CD31-positive endothelial cells.

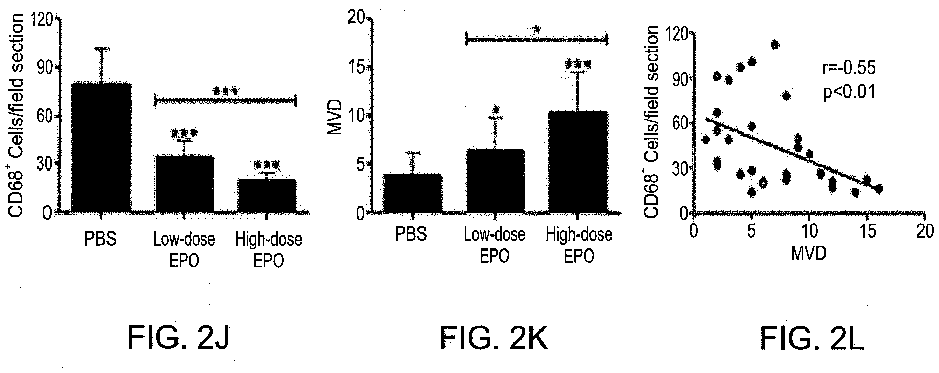

FIGs. 2J-L are graphs depicting the effect of EPO on inflammatory response and MVD in the fat grafts after transplantation. Figure 2J is a bar graph showing that EPO treatment decreases the severity of the inflammatory responses in the fat grafts. Figure 2K is a bar graph showing that EPO treatment increases microvascular density (MVD) in a dose-dependent manner. Each bar represents the mean MVD ± SD from five regions of interest in each fat graft from each treatment group at the end of the 15-week study period. *P<0.05, ***P<0.001, and is the significance of the difference between either the low dose or high dose EPO-treated fat grafts and the PBS-treated grafts. Scale bar: 50 µm. Figure 2L is a line graph showing the negative correlation of MVD to the extent of macrophage infiltration in the fat grafts.

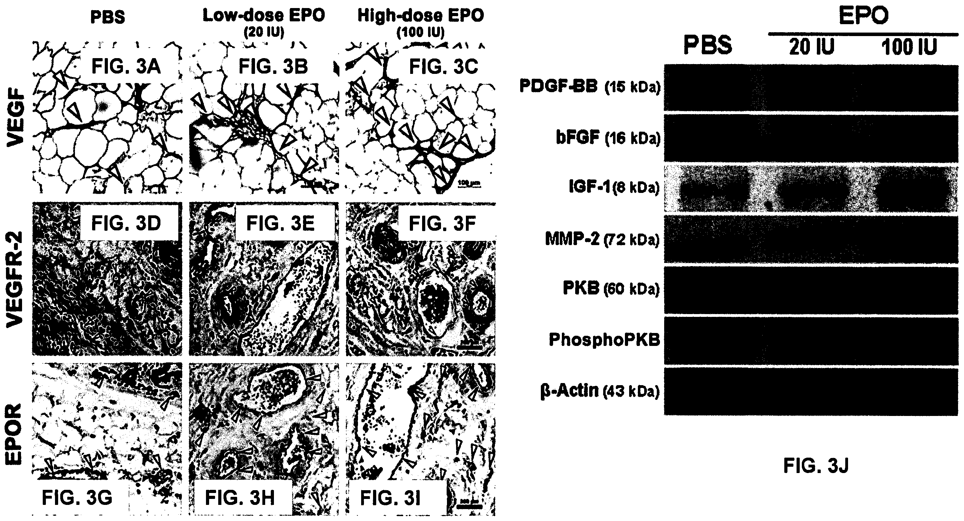

FIGs 3A-J depict the effect of EPO on the expression levels of angiogenic growth factors in the fat grafts. The fat grafts from the three different groups of mice were treated with either PBS (100 µl), 20 IU EPO/100 µl PBS (low-dose), or 100 IU EPO/100 µl PBS (high-dose) on the day of the fat injection, and the treatments were repeated every three days for 18 days. Figures 3A-I are representative histological micrographs of PBS-, and low-dose- and high-dose-EPO treated fat grafts (as indicated) presenting VEGF expression (Figures 3A-C), VEGFR-2 expression (Figures 3D-F) and EPOR expression (Figures 3G-I). Figure 3J is a photograph showing representative western blots of the expression levels of the angiogenic factors in the PBS- and EPO-treated fat grafts at the end of the 15-week study period. bFGF: basic fibroblast growth factor; IGF-1: insulin-like growth factor-1; PDGF-BB: platelet-derived growth factor-BB; MMP-2: matrix metalloproteinase-2; PKB: protein kinase B; phosphoPKB: phosphorylated PKB.

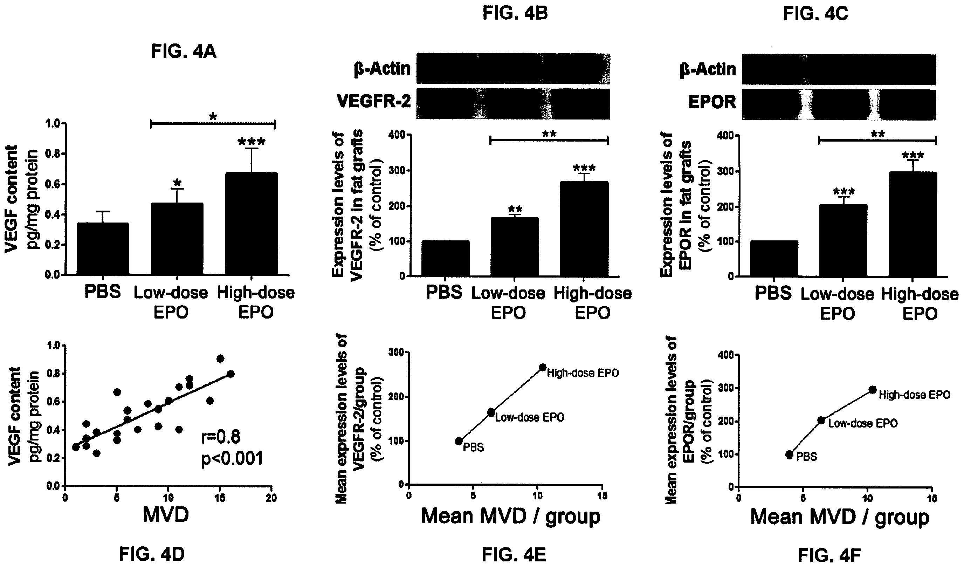

FIGs. 4A-F depict the effect of erythropoietin (EPO) on the expression levels of angiogenic growth factors in the fat grafts. Following implantation into three groups of mice, fat grafts were treated with either PBS (100 µl), 20 IU EPO/100 µl PBS (low-dose), or 100 IU EPO/100 µl PBS (high-dose) on the day of fat injection and repeatedly every three days for a total of 18 days. The graphs represent the mean vascular endothelial growth factor (VEGF) content (Figure 4A), the mean VEGFR-2 expression (Figure 4B) and the mean EPOR expression (Figure 4C) ± SD in the fat grafts in each treatment group. Figures 4D-F show the correlation between VEGF and MVD (Figure 4D), and between mean VEGFR-2 (Figure 4E) and EPOR (Figure 4F) expression and mean MVD in each group. **P* < 0.05, ***P* < 0.01, ****P* < 0.001 for the difference between either the low-dose- or the high-dose EPO-treated fat grafts and the PBS-treated grafts. Scale bar: 200 µm.

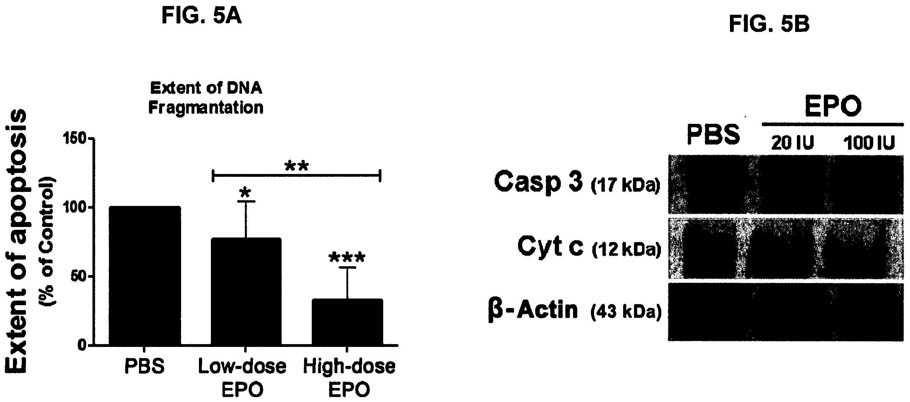

FIGs. 5A-B depict the effect of erythropoietin (EPO) on the extent of apoptosis in the fat grafts. PBS (100 µl), 20 IU EPO/100 µl PBS (low-dose) or 100 IU EPO/100 µl PBS (high-dose) were injected into fat grafts following implantation of the fat grafts into three different groups of mice, this treatment was repeated every three days for 18 days. Figure 5A shows the extent of apoptosis as was measured by TUNEL assay and is expressed as a percentage of the presence of apoptosis in the PBS-treated fat grafts. Each bar represents the mean extent of apoptosis ± SD in the fat graft, in each treatment group, at the end of the 15-week study period. *P<0.05, **P<0.01, ***P<0.001, and is the significance of the difference between either the low dose or high dose EPO-treated fat grafts and the PBS-treated grafts). Figure 5B shows representative western blots of the expression levels of caspase 3 (Casp 3) and cytochrome c (Cyt c) in the PBS- and EPO-treated fat grafts at the end of the 15-week study period.

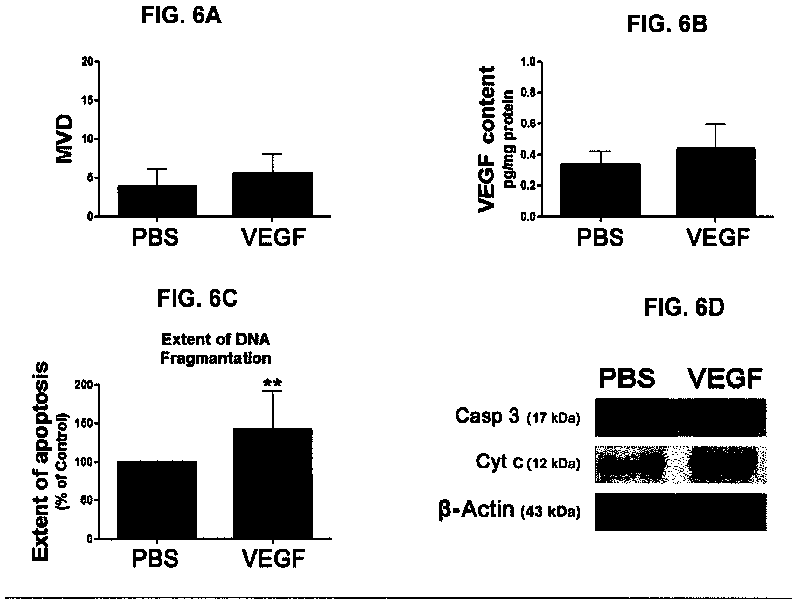

FIGs. 6A-D depict the effect of vascular endothelial growth factor (VEGF) on microvascular density (MVD) and the extent of apoptosis in the fat grafts. PBS (100 µl) or vascular endothelial growth factor (VEGF, 200 ng VEGF/100 µl PBS) were injected into the fat grafts on the day of fat injection into two different groups of mice and then repeatedly every three days for 18 days. Figure 6A is a bar graph showing the mean microvascular density (MVD) ± SD from five regions of interest in each slide (slides were prepared from the harvested fat grafts of each treatment group at the end of the 15-week study period). Figure 6B is a bar graph showing the mean VEGF content ± SD in the harvested fat grafts in each treatment group at the end of the 15-week study period. Figure 6C is a bar graph showing the extent of apoptosis as was measured by TUNEL assay. The results are expressed as a percentage of the extent of apoptosis in the PBS-treated fat grafts. Each bar represents the mean extent of apoptosis ± SD in the fat graft in each treatment group at the end of the 15-week study period. **P<0.01, and is the significance of the difference between the VEGF-treated fat grafts and the PBS-treated grafts. Figure 6D is a photograph showing representative western blots of the expression levels of caspase 3 (Casp 3) and cytochrome c (Cyt c) in the PBS- and VEGF-treated fat grafts at the end of the 15-week study period.

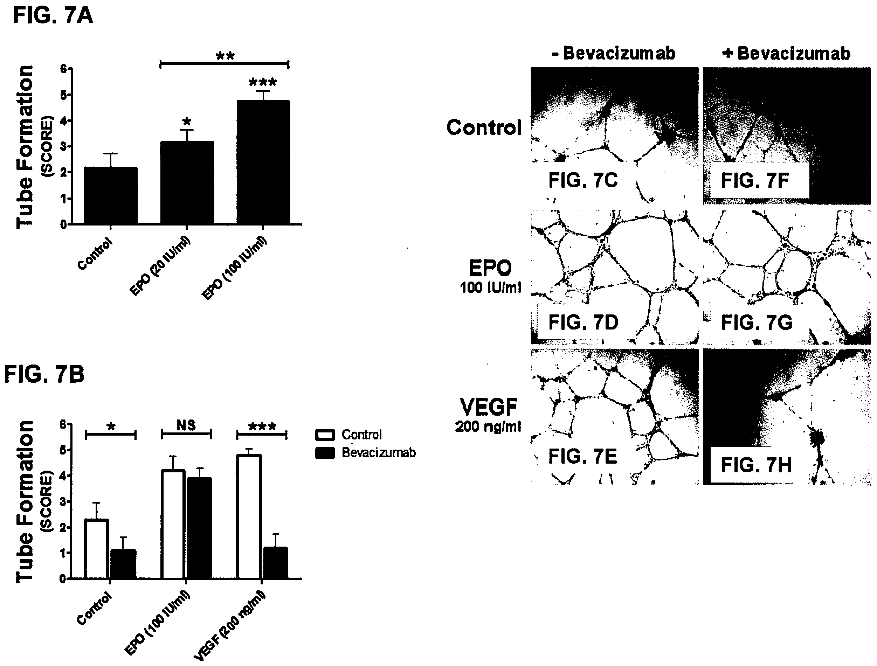

FIG. 7A depict the effect of erythropoietin (EPO) on human umbilical vein endothelial cells (HUVECs) tube formation in matrigel. Human umbilical vein endothelial cells (HUVECs) were treated with 20 IU/ml or 100 IU/ml EPO for 48 hours after plating the cells on matrigel. The extent of HUVEC tube formation on matrigel was assessed after 24 hours under a light microscope at 10 x magnification. The tubular structures were graded semiquantitatively on a scale of 0 to 5 by evaluation of the relative presence and stages of formation of tubes on the matrigel: 0 = well separated individual cells, 1 = cells had begun to migrate and align themselves, 2 = visible capillary tubes and no sprouting, 3 = visible sprouting of new capillary tubes, 4 = early formation of closed polygons, 5 = development of complex mesh-like structures. Each bar represents the mean grade of tube formation ± SD in the matrigel. *P<0.05, **P<0.01 and ***P<0.001.

FIGs. 7B-H depict the effect of EPO or VEGF on human umbilical vein endothelial cells (HUVECs) tube formation in matrigel. HUVECs were treated with 100 IU/ml EPO or 200 ng/100µl VEGF in the absence or presence of 0.25 mg/ml bevacizumab for 48 hours after plating the cells on matrigel. The extent of HUVEC tube formation on matrigel was assessed after 24 hours under a light microscope at 10 x magnification. The tubular structures were graded semiquantitatively on a scale of 0 to 5 by evaluation of the relative presence and stages of formation of tubes on the matrigel: 0 = well separated individual cells, 1 = cells had begun to migrate and align themselves, 2 = visible capillary tubes and no sprouting, 3 = visible sprouting of new capillary tubes, 4 = early formation of closed polygons, 5 = development of complex mesh-like structures. Figure 7B, the white bars represent the mean grade of tube formation ± SD in the matrigel of untreated HUVECs, VEGF- or EPO-treated HUVECs. The black bars represent the mean grade of tube formation ± SD in the matrigel of untreated HUVECs, VEGF- or EPO-treated HUVECs that were exposed to bevacizumab. *P<0.05 and ***P<0.001, and is the significance of the difference between HUVECs that were or not exposed to bevacizumab. NS = not significantly different. Figure 7C depicts untreated HUVECs on matrigel; Figure 7D depicts EPO-treated HUVECs after 24 hours of plating; Figure 7E depicts VEGF-treated HUVECs after 24 hours of plating; Figure 7F depicts untreated HUVECs with bevacizumab; Figure 7G depicts EPO-treated HUVECs after 24 hours of plating with bevacizumab; Figure 7H depicts VEGF-treated HUVECs after 24 hours of plating with bevacizumab.

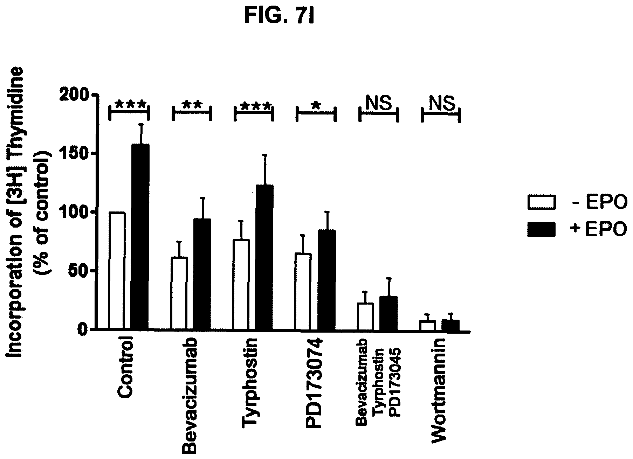

FIG. 7 I depicts the effect of EPO or VEGF on human umbilical vein endothelial cells (HUVECs) tube formation in matrigel. Cultured HUVECs were treated with or without 100 IU/ml EPO in the presence of either bevacizumab, PD173074, or tyrphostin, a combination of bevacizumab, PD173074 and tyrphostin, or in the presence of wortmannin. Proliferation of HUVECs was measured by incorporation of [³H]- thymidine to DNA. Duplicate cell counts were averaged for 3 experiments and the data were expressed as the percentage of control. **P*<0.05, ***P*<0.01 and ****P*<0.001 for the difference between untreated, or EPO- treated HUVECs that were exposed to bevacizumab, PD173074, tyrphostin or wortmannin. NS = not significantly different.

### DESCRIPTION OF SPECIFIC EMBODIMENTS OF THE INVENTION

The principles and operation of the present invention may be better understood with reference to the drawings and accompanying descriptions.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

While reducing the present invention to practice, the present inventor has uncovered that treating engrafted fat tissue with Erythropoietin (EPO) stimulates the release of several angiogenic factors (e.g. VEGF), promotes angiogenesis of the fat tissue and prevents apoptosis of fat graft cells. Moreover, the present inventor has shown that treating the fat grafts with EPO leads to long-term survival of the grafted fat cells. Taken together the present teachings portray a therapeutic value for Erythropoietin and suggest the use of same in transplantation of fat tissue.

As is shown hereinbelow and in the Examples section which follows, the present inventor has uncovered through laborious experimentation that EPO is desirable for promoting fat tissue engraftment. The present inventor has specifically shown that engrafted fat tissue treated with EPO displayed higher weight and volume 15 weeks after fat implantation (Figures 1 A-C and Table 2). The extent of tissue integration was higher in fat tissues treated with EPO while the extent of cyst formation and fibrosis was lower in these tissues (Figures 2A-C and Table 3). Moreover, the EPO treated fat tissues showed high microvascular density (MVD), well vascularized areas with increased expression of CD31 and numerous endothelial islets (Figures 2G-I and 2K) and showed a lower inflammatory response after transplantation (Figures 2D-F and 2J). EPO treatment also lead to a dose-dependent decrease in apoptosis of fat cells (Figure 5 A) while increasing the expression of the angiogenic factors VEGF, bFGF, IGF-1, PDGF-BB, MMP-2 PKB and phosphoPKB (Figures 3J and 4A) and increasing both tissue VEGFR-2 and EPOR expression (Figures 3D-I and 4B-C) in these cells. Taken together, these results substantiate the value of EPO in promoting fat cell engraftment in transplantation procedures.

Thus, according to one aspect of the present invention there is provided
Erythropoietin for use in enhancing implanted fat cell survival for reconstruction surgery in a subject in need thereof, wherein a dose of Erythropoietin for local administration ranges between 1-1000 IU per injection per 1,000,000 fat cells or wherein the dose of Erythropoietin for systemic administration ranges between 10 - 7500 IU per kg body weight.

The terms "fat cell" or "fat cells" as used herein refer to any cell or group of cells composed in a fat tissue, including for example, lipocytes, adipocytes, adipocyte precursors including pre-adipocytes and mesenchymal stem cells. It will be appreciated that according to the present teachings, the fat cells may be dispersed or may be comprised in a tissue.

The number of fat cells may vary over a wide range and one of ordinary skill in the art will recognize that this number will vary depending upon the type and size of the area to be treated according to the use of this invention, the relative degree of vascularization of the area to be treated, the age of the subject to be treated and the relative viability of the fat cells available for transplantation. It will be appreciated that the number of fat cells transplanted may be adjusted according to the procedure used, the site of injection and the relative vascularization of the site to be injected. One of ordinary skill in the art will recognize that certain conditions may necessitate the adjustment of the fat cell numbers outside of the below described ranges. According to some embodiments of the present invention, the number of fat cells for transplantation range from about 10,000 to about 10,000,000 fat cells per 1 ml. According to another embodiment 0.01 - 2000 mls of fat tissue are transplanted. It will be appreciated that the subject may be administered a single transplantation or several transplantations (e.g. about 2, 5, 10, 20, 50, 100 or more transplantation procedures), as described in further detail hereinbelow.

The phrase "fat cell survival" as used herein refers to the ability of the fat cells to remain viable and intact following engraftment thereof. Preferably, the fat cells survive for a period of a few days, a few weeks, a few months or a few years following engraftment thereof.

As used herein, the term "enhancing" in respect to fat cell survival refers to a process of increasing the life span of fat cells in the fat graft and/or decreasing the number of fat cells which undergo resorption, apoptosis or cell death within the fat graft. Thus in some embodiments of the present invention, enhancing refers to at least about 10 %, 20 %, 50 %, 80 %, 90 % increase in viable fat cells and/or at least about 10 %, 20 %, 50 %, 80 %, 90 % arrest in fat cell death. Those of skill in the art will understand that various methodologies and assays can be used to assess cell viability, and similarly, various methodologies and assays may be used to assess cell death or cell apoptosis (e.g. FACS analysis, terminal deoxyuridine triphosphate nick end labeling (TUNEL) assay, cell viability assays e.g. MultiTox Assays).

As mentioned, enhancing fat cell survival according to the present teachings is achieved by administering to the subject Erythropoietin (EPO).

As used herein the term "Erythropoietin" refers to a mammalian (e.g., human) Erythropoietin protein (interchangeably used with polypeptide) or mimetics thereof such as set forth in GenBank Accession No. NP_000790. Erythropoietin may be synthesized using recombinant DNA techniques or solid phase technology. Erythropoietin is also commercially available (e.g., Cytolab/Peprotech, Rehovot, Israel; Arenesp, Amgen, Thousand Oaks, CA, USA; and Epogen, Amgen, Thousand Oaks, CA, USA, Bristol-Myers Squibb, Roche and Sanofi-Aventis). Erythropoietin may be used as an entire glycoprotein or as only a protein subunit devoid of the bound sugar. Since the Erythropoietin of the present invention is used for clinical applications, it is preferably sterile or may be purified of possible contaminating factors (e.g., bacteria or bacterial components, such as by filter).

Typical subjects that may be treated according to this aspect of the present invention include mammals such as human beings or domesticated animals including, but not limited to, horses (i.e. equine), cattle, goat, sheep, pig, dog, cat, camel, alpaca, llama and yak, male or female, at any age that is in need of fat transplantation.

In general, fat transplantation may be used to treat any soft tissue defect, to fill any soft tissue deficit and for augmentation of external and internal surfaces and structures of the body which are missing due to surgery, as a result of aging of a tissue, or due to disease, trauma or an injury. Examples include, but are not limited to, urological surgeries, tumor removal surgeries, reconstructive surgeries and skin surgeries. Likewise, fat transplantation may be used as an alternative to silicone or collagen fillers. Fat transplantation may be used to fill depressions (i.e. areas of the body which are hollow or sunken and lack the cellular substance, body or volume compared to the same area on a normal body) after injury or pursuant to surgical procedures such as cosmetic surgery, including, but not limited to, facelifts, mastectomies or lumpectomies and due to other procedures, as for example, removal of cancerous tissues, especially tumors at or near the skin of the subject. Fat transplantation may also be used in numerous other applications, including urological procedures involving the buildup of weak or damaged structural tissue, in treatment of wrinkles, burns, skin conditions, skin maladies and wounds and to augment areas of the body, such as the buttocks, biceps, triceps muscles, calf muscles, breasts, hands and penis. Furthermore, fat transplantation may be used to treat congenital malformations such as Hemifacial microsomia and acquired diseases such as Romberg's lipodystrophy and Acquired immune deficiency syndrome (AIDS).

It will be appreciated that the fat cells may be obtained from the body of a subject and used in an autologous fashion (i.e. transplanted into the same subject from which the fat cells were obtained). In cases where an autologous fat transplant is carried out, the autologous fat cells are typically taken from a subject to fill in depressions or soft tissue deficits in the body of the same subject in an area of the body other than that site from which the fat cells were removed.

Alternatively, the fat cells may be obtained from one subject (a "donor") and transplanted into a different individual (a "recipient") in a non-autologous fashion. In cases where a non-autologous fat transplant is carried out, the fat cells may be obtained from a subject of the same species as the recipient subject (i.e. allogeneic fat cells as for example from a human donor to a human recipient) or from a different species (i.e. xenogeneic cells as for example from a porcine donor to a human recipient). Such methods are well known to one of ordinary skill in the art. According to an embodiment of the present invention, the non-autologous cell is obtained from a mammal.

According to the present teachings, fat cells are generally obtained by removing same (e.g. by suctioning) from subcutaneous fat layers in the area of the stomach, legs or other areas where significant fat cells may be found. Preferably the fat cells in the present invention are substantially free of unrelated cells such as erythrocytes, other blood cells, fibroblasts and other cells which may contaminate the fat cells. Furthermore, as the fat cells are used for transplantation, these cells are kept in a sterile environment until used for transplantation.

It will be appreciated that the fat cells may be further separated from other components which may be found in the aspirated fat, such as, for example, triglycerides, lysozomes, other cellular fragments, blood components, blood cells and large connective tissue fragments, among other less desirable components, before use. Any methods known in the art may be used to separate the fat cells from these other components, but preferably, at least one centrifugation step is employed.

Fat cells may be immediately implanted into a subject. Preferably the fat cells may be implanted within 30 minutes, within an hour, within two hours, within three hours, within four hours or within one day of collection (see e.g. Example 1, of the examples section which follows). It will be appreciated that the fat cells in the present invention may be preserved for longer periods of time prior to translation in, for example, by freezing in liquid nitrogen.

Implanting the fat cells may be carried out by any method known in the art, such as for example, by injection thereof into the desired location (as described in detail in Example 1, hereinbelow), by microsurgery and by surgery in cases were a large amount of fat cells or fat tissue is being transplanted.

Following implantation of the fat cells, the subject may be administered Erythropoietin.

It will be appreciated that Erythropoietin may be administered via a systemic administration or via a local administration.

As used herein the phrase "systemic administration" refers to oral, intravenous, intraperitoneal and intramuscular administration of Erythropoietin.

As used herein the phrase "local administering" refers to applying the Erythropoietin directly to the implanted fat cells or in close proximity to the implanted fat cells. Erythropoietin may be directly administered to the transplanted fat cells via injection.

It will be appreciated that according to the teachings of the present invention the contemplated dose of Erythropoietin applied for local administration (e.g. for direct injection into the implanted fat cells) ranges between 1-1000 IU per injection per 1,000,000 fat cells for local administration. Likewise, the dose of Erythropoietin for systemic administration ranges between 10 - 7500 IU per kg body weight for systemic administration. The dose of Erythropoietin selected for treatment depends on the number and concentration of fat cells, the subject being treated and the location of the graft.

It will be appreciated that when mimetics compositions are used the dosages of Erythropoietin should be calibrated such as according to the molar value. Such a calibration is a routine calculation for those of ordinary skill in the art.

Administration of Erythropoietin is typically effected immediately following implantation of the fat cells. Thus, Erythropoietin is administered to the subject within a few minutes or within a few hours of implantation. According to a specific embodiment, Erythropoietin is administered to the subject starting from the first day of fat cell transplantation and is continuously administered until the fat cells have been integrated and vascularized in the subject (e.g. for at least 5- 50 days).

According to a specific embodiment, the present invention contemplates treating fat cells with Erythropoietin prior to implantation thereof. This may be in addition to administration of Erythropoietin following implantation or instead of administration of Erythropoietin following implantation. Treatment of the fat cells may be carried out by any method known to one of ordinary skill in the art as for example by ex vivo contacting the fat cells with Erythropoietin in a tissue culture plate or by injection of Erythropoietin directly into the fat tissue. Alternatively, fat cells may be exposed to Erythropoietin prior to removal from the donor.

Concentrations of Erythropoietin for treating fat cells prior to transplantation are in a dose between 1-1000 IU per injection per 1,000,000 fat cells.

The subject being treated prior to implantation may continue to receive Erythropoietin following implantation of the fat cells as depicted in detail hereinabove.

Erythropoietin can be administered to the subject per se or as a pharmaceutical composition. In addition, the fat cells in the present invention can be administered per se or as part of a pharmaceutical composition.

As used herein a "pharmaceutical composition" refers to a preparation of the active ingredients described herein with other chemical components such as physiologically suitable carriers and excipients. The purpose of the composition is to facilitate administration of the active ingredients (e.g., Erythropoietin) to the subject.

As used herein the term "active ingredient" refers to Erythropoietin or the fat cells themselves accountable for the intended biological effect (*i.e.,* enhancing fat cell survival).

Hereinafter, the phrases "physiologically acceptable carrier" and "pharmaceutically acceptable carrier" which may be interchangeably used refer to a carrier or a diluent that does not cause significant irritation to the subject and does not abrogate the biological activity and properties of the administered active ingredients. An adjuvant is included under these phrases.

Herein, the term "excipient" refers to an inert substance added to the composition (pharmaceutical composition) to further facilitate administration of an active ingredient in the present invention.

Techniques for formulation and administration of drugs may be found in "Remington's Pharmaceutical Sciences," Mack Publishing Co., Easton, PA, latest edition.

As mentioned hereinabove, suitable routes of administration of Erythropoietin may, for example, include a systemic manner including oral, rectal, transmucosal, especially transnasal, intestinal or parenteral delivery, including intramuscular, subcutaneous and intramedullary injections as well as intrathecal, direct intraventricular, intravenous, intraperitoneal, intramuscular, intranasal, or intraocular injections.

Alternately, one may administer the pharmaceutical composition comprising Erythropoietin in a local rather than systemic manner, for example, via injection of the pharmaceutical composition directly into the fat cell implant region of a patient, or via application of the compositions directly into a tissue region in proximity to the fat cell implant of a patient. Suitable routes of administration of the compositions may, for example, include topical (e.g., to a keratinous tissue, such as the skin, scalp) and mucosal (e.g., oral, vaginal, eye) administrations.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations which can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continues infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of the present invention may also be formulated in rectal compositions such as suppositories or retention enemas, using, e.g., conventional suppository bases such as cocoa butter or other glycerides.

Pharmaceutical compositions suitable for use in context of the present invention include compositions wherein the active ingredients are contained in an amount effective to achieve the intended purpose. A therapeutically effective amount means an amount of active ingredients (e.g. Erythropoietin) at a dose of about 1-1000 IU per injection per 1,000,000 fat cells, effective in enhancing fat cell survival.

Determination of a therapeutically effective amount is well within the capability of those skilled in the art, especially in light of the detailed disclosure provided herein.

For any preparation used in the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to levels of the active ingredient which are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

An animal model which can be used according to the present teachings to assess the biological effect of the compositions described herein includes SCID mice (as described in detail in the Examples section below).

Depending on the severity of the condition being treated, the number of fat cells being implanted and the responsiveness of the subject to the treatment, dosing can be of a single or a plurality of administrations, with course of treatment lasting from several days to several weeks or months or until cure is effected or until ample fat tissue has been endured.

According to some embodiments of the present invention, Erythropoietin compositions are administered once, administered twice, administered three times, administered four times, administered five times, administered six times, administered seven times, administered eight times, administered nine times or administered ten times to the subject in order to enhance fat cell survival. It will be appreciated that if multiple fat cell transplantations are carried out, the number of administrations of Erythropoietin may be vast and may be prolonged for as long as needed (as determined by one of ordinary skill in the art). Preferably, the compositions of the present invention are administered at least once a day. It will be appreciated that the number of administrations can be determined by one of ordinary skill in the art.

The amount of a composition to be administered will, of course, be dependent on the subject being treated, the severity of the affliction, the manner of administration, the judgment of the prescribing physician, etc.

Determination of efficacy of treatment may be determined by measuring the number and viability of the engrafted fat cells (e.g. by ultrasound), measuring the number of apoptotic cells within the graft (e.g. by PCR), and evaluating the vascularization of the transplanted fat cells (e.g. by ultrasound).

Compositions of the present invention may, if desired, be presented in a pack or dispenser device, such as an FDA-approved kit, which may contain one or more unit dosage forms containing the active ingredient. The pack may, for example, comprise metal or plastic foil, such as a blister pack. The pack or dispenser device may be accompanied by instructions for administration. The pack or dispenser device may also be accompanied by a notice in a form prescribed by a governmental agency regulating the manufacture, use, or sale of pharmaceuticals, which notice is reflective of approval by the agency of the form of the compositions for human or veterinary administration. Such notice, for example, may include labeling approved by the U.S. Food and Drug Administration for prescription drugs or of an approved product insert. Compositions comprising a preparation of the invention formulated in a pharmaceutically acceptable carrier may also be prepared, placed in an appropriate container, and labeled for treatment of an indicated condition, as further detailed above.

Since the compositions of the present invention are utilized *in vivo,* the compositions are preferably of high purity and substantially free of potentially harmful contaminants, e.g., at least National Food (NF) grade, generally at least analytical grade, and preferably at least pharmaceutical grade. To the extent that a given compound must be synthesized prior to use, such synthesis or subsequent purification shall preferably result in a product that is substantially free of any potentially contaminating toxic agents that may have been used during the synthesis or purification procedures.

Additional factors may be incorporated into the compositions of the present invention (*i.e.,* Erythropoietin described hereinabove) to enhance fat cell survival. These include, but are not limited to, extracellular matrix components (e.g. vitronectin, laminin, collagen, elastin), growth factors (e.g. FGF 1, FGF 2, IGF 1, IGF 2, PDGF, EGF, KGF, HGF, VEGF, GM-CSF, CSF, G-CSF, TGF alpha, TGF beta, NGF and ECGF), hypoxia inducible factors (e.g. HIF-1 alpha and beta and HIF-2), hormones (e.g., insulin, growth hormone (GH), CRH, Leptin, Prolactin and TSH), angiogenic factors (e.g., angiogenin and angiopoietin), coagulation and anticoagulation factors [e.g., Factor I, Factor XIII, tissue factor, calcium, vWF, protein C, protein S, protein Z, fibronectin, antithrombin, heparin, plasminogen, low molecular weight heparin (Clixan), high molecular weight kininogen (HMWK), prekallikrein, plasminogen activator inhibitor-1 (PAI1), plasminogen activator inhibitor-2 (PAI2), urokinase, thrombomoduline, tissue plasminogen activator (tPA), alpha 2-antiplasmin and Protein Z-related protease inhibitor (ZPI)], cytokines (IL-1 alpha, IL-1 beta, IL-2, IL-3, IL-4, IL-5, IL-6, IL-7, IL-8, IL-9, IL-10, IL-11, IL-12, IL-13 and INF-alpha, INF, beta, and INF-gamma), chemokines (e.g., MCP-1 or CCL2), enzymes (e.g. endoglycosidases, exoglycosidases, endonucleases, exonucleases, peptidases, lipases, oxidases, decarboxylases, hydrases, chondroitinase, chondroitinase ABC, chondroitinase AC, hyaluronidase, keratanase, heparanases, heparanase splice variance, collagenase, trypsin, catalases), neurotransmitters (e.g., acetylcholine and monoamines), neuropeptides (e.g. substance P), vitamins (e.g., D-biotin, Choline Chloride, Folic acid, Myo-inositol, Niacinamide, D-Pantothenic acid, Calcium salts, Pyridoxal.HCl, Pyrodixine.HCl, Riboflavin, Thiamine.HCl, Vitamin B12, vitamin E, vitamin C, vitamin D, vitamin B1-6, vitamin K, vitamin A and vitamin PP), carbohydrates (e.g. Mono/Di/Polysacharides including glucose, mannose, maltose and fructose), ions, chelators (e.g. Fe chelators, Ca chelators), antioxidants (e.g., Vitamin E, Quarcetin, superoxide scavengers, Superoxide dismutase), H2O2 scavengers, free radicals scavengers, Fe scavengers), fatty acids (e.g., Triglycerides, Phospholipids, Cholesterols, free fatty acids and non free fatty acids, fatty alcohol, Linoleic acid, oleic acid and lipoic acid), antibiotics (e.g., Penicillins, Cephalosporins and Tetracyclines), analgesics, anesthetics, antibacterial agents, anti-yeast agents, anti-fungal agents, antiviral agents, pro-biotic agents, anti-protozal agents, anti-pruritic agents, anti-dermatitis agents, anti-emetics, anti-inflammatory agents, anti-hyperkeratolyic agents, antiperspirants, anti-psoriatic agents, anti-seborrheic agents, antihistamine agents, amino acids (e.g., essential and non essential (from A-Z) especially glutamine and arginine), salts (e.g., prurivat salts and sulfate salts), sulfates (e.g. Calcium Sulfate), steroids (e.g., androgens, estrogens, progestagens, glucocorticoids and mineralocorticoids), catecholamines (e.g., Epinephrine and Nor-epinephrine), Nucleosides and Nucleotides (e.g., Purins and Pyrimidines), Prostaglandins (e.g. Prostaglandin E2), Leucotriens, Erythropoietins (e.g. Thrombopoietin), Proteoglycans (e.g. Heparan sulfate, keratan sulfate), Hydroxyapatites [e.g. Hydroxyapatite (Ca₁₀(PO₄)₆(OH)₂)], Haptoglobins (Hp1-1, Hp2-2 and Hp1-2), Superoxide dismutases (e.g. SOD 1/2/3), Nitric Oxides, Nitric Oxide donors (e.g. nitroprusside, Sigma Aldrich, St. Louis, MO, USA, Glutathione peroxidases, Hydrating compounds (e.g. vasopressin), cells (e.g. Platelets), cell medium (e.g. M199, DMEM/F12, RPMI, Iscovs), serum (e.g. human serum, fetal calf serum, , fetal bovine serum), buffers (e.g., HEPES, Sodium Bicarbonate), detergents (e.g., Tween), disinfectants, herbs, fruit extracts, vegetable extracts (e.g. cabbage, cucumber), flower extracts, plant extracts, flavinoids (e.g. pomegranate juice), spices, leafs (e.g. Green tea, Chamomile), Polyphenols (e.g. Red Wine), honey, lectins, microparticles, nanoparticles (lyposomes), micelles, calcium carbonate (CaCO3, e.g. precipitated calcium carbonate, ground/pulverized calcium carbonate, albacar, PCC, GCC), calcite, limestone, crushed marble, ground limestone, lime, chalk (e.g. whiting chalk, champagne chalk, french chalk) and co factors such as BH4 (tetrahydrobiobterine).

The present composition may also contain ingredients, substances, elements and materials containing, hydrogen, alkyl groups, aryl groups, halo groups, hydroxy groups, alkoxy groups, alkylamino groups, dialkylamino groups, acyl groups, carboxyl groups, carboamido groups, sulfonamide groups, aminoacyl groups, amide groups, amine groups, nitro groups, organo selenium compounds, hydrocarbons, and cyclic hydrocarbons.

The present composition may be combined with substances such as benzol peroxide, vasoconstrictors, vasodilatators, salicylic acid, retinoic acid, azelaic acid, lactic acid, glycolic acid, pyreuric acid, tannins, benzlidenecamphor and derivatives thereof, alpha hydroxyis, surfactants.

Compositions of some embodiments of the present invention may be bioconjugated to polyethylenglycol (e.g. PEG, SE-PEG) which preserves the stability (e.g., against protease activities) and/or solubility (e.g., within a biological fluid such as blood, digestive fluid) of the active ingredients (e.g. Erythropoietin) while preserving their biological activity and prolonging its half-life.

It will be appreciated that compositions of the present invention can be used in combination with other currently practiced therapies for fat cell transplantation as, without being limited to, treatment of the subject with growth factors, transplantation of the fat cells on scaffolds or transplantation of the fat cells on polyester beads.

As mentioned, fat cells of the present invention can be derived from either autologous sources or from non-autologous sources (e.g. allogeneic or xenogeneic). Since non-autologous cells are likely to induce an immune reaction when administered to the body several approaches have been developed to reduce the likelihood of rejection of non-autologous cells. These include either suppressing the recipient immune system or encapsulating the non-autologous cells or tissues in immunoisolating, semipermeable membranes before transplantation.

Encapsulation techniques are generally classified as microencapsulation, involving small spherical vehicles and macroencapsulation, involving larger flat-sheet and hollow-fiber membranes (Uludag, H. et al. Technology of mammalian cell encapsulation. Adv Drug Deliv Rev. 2000; 42: 29-64).

Methods of preparing microcapsules are known in the arts and include for example those disclosed by Lu MZ, et al., Cell encapsulation with alginate and alpha-phenoxycinnamylidene-acetylated poly(allylamine). Biotechnol Bioeng. 2000, 70: 479-83, Chang TM and Prakash S. Procedures for microencapsulation of enzymes, cells and genetically engineered microorganisms. Mol Biotechnol. 2001, 17: 249-60, and Lu MZ, et al., A novel cell encapsulation method using photosensitive poly(allylamine alpha-cyanocinnamylideneacetate). J Microencapsul. 2000, 17: 245-51.

For example, microcapsules are prepared by complexing modified collagen with a ter-polymer shell of 2-hydroxyethyl methylacrylate (HEMA), methacrylic acid (MAA) and methyl methacrylate (MMA), resulting in a capsule thickness of 2-5 µm. Such microcapsules can be further encapsulated with additional 2-5 µm ter-polymer shells in order to impart a negatively charged smooth surface and to minimize plasma protein absorption (Chia, S.M. et al. Multi-layered microcapsules for cell encapsulation Biomaterials. 2002 23: 849-56).

Other microcapsules are based on alginate, a marine polysaccharide (Sambanis, A. Encapsulated islets in diabetes treatment. Diabetes Thechnol. Ther. 2003, 5: 665-8) or its derivatives. For example, microcapsules can be prepared by the polyelectrolyte complexation between the polyanions sodium alginate and sodium cellulose sulphate with the polycation poly(methylene-co-guanidine) hydrochloride in the presence of calcium chloride.

It will be appreciated that cell encapsulation is improved when smaller capsules are used. Thus, the quality control, mechanical stability, diffusion properties, and in vitro activities of encapsulated cells improved when the capsule size was reduced from 1 mm to 400 µm (Canaple L. et al., Improving cell encapsulation through size control. J Biomater Sci Polym Ed. 2002;13: 783-96). Moreover, nanoporous biocapsules with well-controlled pore size as small as 7 nm, tailored surface chemistries and precise microarchitectures were found to successfully immunoisolate microenvironments for cells (Williams D. Small is beautiful: microparticle and nanoparticle technology in medical devices. Med Device Technol. 1999, 10: 6-9; Desai, T.A. Microfabrication technology for pancreatic cell encapsulation. Expert Opin Biol Ther. 2002, 2: 633-46).

As mentioned above, in order to facilitate engraftment of non-autologous fat cells, the method of the present invention may further advantageously comprise conditioning the subject with an immunosuppressive regimen prior to, concomitantly with, or following transplantation of the fat cells.

According to a specific embodiment, the methods of the present invention require a reduced immunosuppressive regimen as compared to a subject not treated with Erythropoietin.

Examples of suitable types of immunosuppressive regimens include administration of immunosuppressive drugs and/or immunosuppressive irradiation.

Ample guidance for selecting and administering suitable immunosuppressive regimens for transplantation is provided in the literature of the art (for example, refer to: Kirkpatrick CH. and Rowlands DT Jr., 1992. JAMA. 268, 2952; Higgins RM. et al., 1996. Lancet 348, 1208; Suthanthiran M. and Strom TB., 1996. New Engl. J. Med. 331, 365; Midthun DE. et al., 1997. Mayo Clin Proc. 72, 175; Morrison VA. et al., 1994. Am J Med. 97, 14; Hanto DW., 1995. Annu Rev Med. 46, 381; Senderowicz AM. et al., 1997. Ann Intern Med. 126, 882; Vincenti F. et al., 1998. New Engl. J. Med. 338, 161; Dantal J. et al. 1998. Lancet 351, 623).

Preferably, the immunosuppressive regimen consists of administering at least one immunosuppressant agent to the subject.

Examples of immunosuppressive agents include, but are not limited to, methotrexate, cyclophosphamide, cyclosporine, cyclosporin A, chloroquine, hydroxychloroquine, sulfasalazine (sulphasalazopyrine), gold salts, D-penicillamine, leflunomide, azathioprine, anakinra, infliximab (REMICADE), etanercept, TNF.alpha. blockers, a biological agent that targets an inflammatory cytokine, and Non-Steroidal Anti-Inflammatory Drug (NSAIDs). Examples of NSAIDs include, but are not limited to acetyl salicylic acid, choline magnesium salicylate, diflunisal, magnesium salicylate, salsalate, sodium salicylate, diclofenac, etodolac, fenoprofen, flurbiprofen, indomethacin, ketoprofen, ketorolac, meclofenamate, naproxen, nabumetone, phenylbutazone, piroxicam, sulindac, tolmetin, acetaminophen, ibuprofen, Cox-2 inhibitors and tramadol. These agents may be administered individually or in combination.

According to another embodiment, the methods of the present invention require a reduced anti-inflammatory treatment [e.g. anti-inflammatory drugs such as steroids, non-steroidal anti-inflammatory drugs or immune selective anti-inflammatory derivatives (ImSAIDs)] as compared to a subject not treated with Erythropoietin.

As used herein the term "about" refers to ± 10 %.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### EXAMPLE 1

### Comparison between low dose EPO, high dose EPO and VEGF treatment on fat grafts weight and volume

### MATERIALS AND EXPERIMENTAL PROCEDURES

### Isolation and Preparation of Human Fat Tissue

Fat was harvested from the thigh of a 40-year-old woman by suction-assisted lipectomy under general anesthesia. The fat was aspirated under local anesthesia using a 14-gauge blunt cannula, and then processed under sterile conditions for subsequent grafting into nude mice within two hours of its collection according to previously published protocols [Ullmann et al. (2005) Dermatol Surg 31:1304-7; Kurita et al. (2008) Plast Reconstr Surg 121:1033-1041].

### Study Design

Two different animal studies were conducted herein.

The first study comprised 30 seven-week-old female CD-1 nude mice (Harlan, Jerusalem, Israel). These mice were housed in cages in a room with an artificial 12-h light/dark cycle at a constant temperature range (24 ± 2 °C) and relative humidity (55 ± 10%). The mice were acclimated for one week prior to the study, and fed a standard laboratory chow and water ad libitum. The 30 mice were randomly divided into three equal groups and treated as follows: Group 1 mice were injected with 1 ml of human fat and were treated with sterile PBS (control group). Group 2 mice were injected with 1 ml human fat and were treated with 1000 IU/kg EPO (low-dose EPO group). Group 3 mice were injected with 1 ml human fat and were treated with 5000 IU/kg EPO (high-dose EPO group). The fat was injected subcutaneously into the scalp using a 14G needle while the animals were manually restrained. Immediately following fat transplantation, the PBS-treated fat grafts were injected with 100 µl PBS (control group), and the EPO-treated fat grafts were injected with either 20 IU EPO/100 µl PBS (low-dose EPO group) or 100 IU EPO/100 µl PBS (high-dose EPO group) every three days for 18 days (a total of 6 injections). EPO was purchased as an injection ampoule (ARANESP®, Amgen AG, Zug, Switzerland) which contained 150 µg/ml (18,000 IU) of EPO.

The second animal study comprised 20 seven-week-old female CD-1 nude mice, and differed from the first study in that the fat was treated with VEGF (2 µg/ml, Sigma Aldrich, MO, USA) following implantation. 10 mice were injected with fat followed by 200 ng VEGF/100 µl PBS injections every three days for a total of 18 days. The remaining 10 mice made up a second control group that was treated in an identical manner to the control group in the first study. Post-operative analgesics and antibiotics were not administered to the mice in the two experiments.

### Follow-up and Data Collection

The duration of the study period of both experiments was 15 weeks from the start of fat transplantation. During this period, each mouse was weighed; a tail vein blood sample was collected for determination of the red blood cell count, leukocyte count, and platelet count; and for measurement of plasma hemoglobin, VEGF, and EPO concentrations. These measurements were carried out at three different occasions: the day of fat injection, 18 days after the fat injection and at the end of the study period. VEGF and EPO concentrations were determined in homogenates of samples of the fat grafts using commercial enzyme-linked immunosorbent assays (Quantikine® VEGF immunoassay Kit and Quantikine® IVD® Erythropoietin Kit, R&D Systems, MN, USA) in accordance with the manufacturer's instructions.

After 15 weeks, all mice were humanely killed and the fat grafts were carefully dissected from their scalps (Figure 1C). Each fat graft was weighed and the volume of the fat graft was measured by the liquid overflow method as previously described [Ayhan et al. (2001) Aesthetic Plast Surg 25:338-342]. After weight and volume determination, each fat graft was divided into two portions from the middle. One portion was stored at -80 °C for later determination of EPO concentrations, VEGF content, the extent of apoptosis, and the expression levels of the angiogenic factors, namely bFGF, insulin growth factor-1 (IGF-1), PDGF-BB, VEGF receptor-2 (VEGFR-2), EPO receptor (EPOR) and MMP-2, the survival factor PKB and phosphorylated PKB, and the pro-apoptotic factors, namely caspase 3 and cytochrome c. The second portion was placed in 4 % formalin and used for determination of macrophage infiltration, microvascular density (MVD), VEGFR-2 and EPOR localization and for histological examination.

### Statistical Analysis of the Data

Data for each study parameter from the PBS-, VEGF- or EPO-treated fat grafts from each treatment group were pooled, and the results were presented as mean ± standard deviation (SD). The data displayed a normal distribution by the Kolmogorov-Smirnov test. The data from the first experiment was analyzed by ANOVA and the data from the second experiment was analyzed by a Student's *t* test, using a computerized statistical software program (Prism version 5.0, GraphPad Software Inc, CA, USA). Differences were considered statistically significant when *P* ≤ 0.05. Kappa values for intra-examiner repeatability of the blinded evaluations of histological analysis, MVD, and tube formation in matrigel were 0.94, 0.89, and 0.93, respectively.

### RESULTS

All mice in all treatment groups in both experiments completed the 15-week study period. They appeared to be healthy during the course of the study and there was no evidence of cachexia at the end of the study period. There were no significant changes in red blood cell counts, leukocyte counts, platelet counts, plasma hemoglobin and EPO concentrations in mice with either phosphate buffer saline (PBS)-treated or low-dose EPO-treated fat grafts (Table 1, below). The red blood cell counts, leukocyte counts, platelet counts and plasma EPO concentrations, but not the plasma hemoglobin concentrations, were significantly increased in mice treated with high-dose EPO-treated fat grafts (Table 1, below). Eighteen days after transplantation, plasma VEGF concentrations were significantly increased in the two groups of mice with EPO-treated fat grafts. At the end of the 15-week study period, the plasma VEGF concentrations in the two groups of mice with EPO-treated grafts were not significantly different from baseline values and those in mice with PBS-treated fat grafts. At the end of the 15-week study period, EPO concentrations in the PBS- and EPO-treated grafts were not different from the baseline values, however, 18 days after fat injection both the EPO and VEGF values were significantly higher in both the EPO-low and EPO-high treatment groups (Table 1, below).

**Table 1: Effect of EPO treatment on body weight, hematology, plasma and tissue EPO concentrations**

| | **Control (n=10)** | **Low-Dose EPO (n=10)** | **High-Dose EPO (n=10)** |
|---|---|---|---|
| **Initial mice weight (g)** | 26.7±1.1 | 25.9±1.1 | 26.2±1.0 |
| After EPO treatment | 27.3±1.1 | 27.9±1.1 | 28.6±1.2 |
| At week 15 | 28.3±1.1 | 28.8±1.1 | 29.0±1.2 |
| **Initial RBC count (10⁶/mm³)** | 7.8±0.9 | 8.0±1.0 | 7.9±1.2 |
| After EPO treatment | 7.9±0.9 | 8.0±1.1 | 8.9±1.0* |
| At week 15 | 7.8±0.9 | 7.9±1.0 | 8.1±1.2 |
| **Initial leukocyte count (10⁶/mm³)** | 10.8±1.2 | 11.1±1.1 | 10.9±1.2 |
| After EPO treatment | 11.2±1.2 | 11.4±1.1 | 13.1±1.3* |
| At week 15 | 11.0±1.1 | 10.8±1.1 | 11.4±1.2 |
| **Initial platelet count (10³/L)** | 593±54 | 609±63 | 603±72 |
| After EPO treatment | 579±58 | 621±68 | 741±81** |
| At week 15 | 593±54 | 601±57 | 597±64 |
| **Initial hemoglobin concentrations (g/dl)** | 14.4±1.3 | 15.1±1.4 | 15.5±1.4 |
| After EPO treatment | 14.8±1.3 | 15.7±1.4 | 16.4±1.6 |
| At week 15 | 14.8±1.2 | 15.1±1.6 | 14.9±1.5 |
| **Initial plasma EPO concentrations** | 14.3±1.9 | 14.6±1.3 | 14.2±1.7 |

| **(mU/mL)** | | | |
|---|---|---|---|
| After EPO treatment | 13.7±1.4 | 17.6±3.3* | 46.7±8.7*** |
| At week 15 | 14.3±1.7 | 14.2±1.3 | 14.1±1.3 |
| **Initial plasma VEGF** | 38.6±3.9 | 34.8±4.6 | 39.2±4.8 |

| **concentrations (pg/mL)** | | | |
|---|---|---|---|
| After EPO treatment | 37.1±3.8 | 51.5±6.6* | 87±9.2*** |
| At week 15 | 38.0±3.3 | 36.6±4.9 | 37.4±5.3 |
| **Tissue EPO concentrations (mU/mL)** | 0.3±0.1 | 0.3±0.1 | 0.3±0.1 |

| | | | |
|---|---|---|---|
| Of note: Values are presented as mean ± SD; n = number of mice; RBC = red blood cells; EPO = erythropoietin; VEGF = vascular endothelial growth factor. *P<0.05, **P<0.01, ***P<0.001 and is the difference between either the low dose or high dose-treated EPO grafts PBS-treated grafts | | | |

Furthermore, at the end of the 15-week study period a well-defined, subcutaneous lump was observed on the scalp of each mouse (Figures 1A-C). The weights and volumes of the EPO-treated grafts were higher than those of the PBS-treated grafts (Table 2, below). The weights and volumes of the PBS-treated fat grafts in the first experiment were not different from those in PBS- and VEGF-treated grafts in the second experiment (Table 2, below).

**Table 2: Effect of EPO treatment on fat graft weight and volume**

| | **First Experiment** | | | **Second Experiment** | |
|---|---|---|---|---|---|
| | **PBS** | **Low-Dose EPO** | **High-Dose EPO** | **PBS** | **VEGF** |
| | **(n=10)** | **(n=10)** | **(n=10)** | **(n=10)** | **(n=10)** |
| Weight (g) | 0.3±0.1 | 0.5±0.2** | 0.6±0.2*** | 0.32±0.2 | 0.35±0.2 |
| Volume (ml) | 0.3±0.1 | 0.4±0.1** | 0.6±0.1*** | 0.35±0.1 | 0.36±0.2 |

| | | | | | |
|---|---|---|---|---|---|
| Of note: Values are presented as mean ± SD n = number of mice EPO = erythropoietin VEGF = vascular endothelial growth factor **P<0.01, ***P<0.001, and is the significance of the difference between either the low dose or high dose EPO-treated fat grafts and the PBS-treated grafts. | | | | | |

### EXAMPLE 2

### Histological evaluation of the fat grafts and the effect of EPO on inflammatory response in fat grafts

### MATERIALS AND EXPERIMENTAL PROCEDURES

### Isolation and Preparation of Human Fat Tissue

As described in Example 1, hereinabove.

### Study Design

As described in Example 1, hereinabove.

### Histological Analysis

Histological slides of the formalin-maintained samples were prepared and then stained with hematoxylin and eosin using standard procedures. Immunohistochemistry was performed using rabbit monoclonal antibodies against tissue CD31, VEGFR-2 and EPOR, and goat polyclonal IgG against VEGF (R&D Systems, Minneapolis MN, USA), and CD68 (Dako, Glostrup, Denmark). The paraffin-embedded fat graft sections were incubated with the antibodies overnight at room temperature followed by incubation with appropriate secondary antibodies [Li et al. (2005) J Cell Biochem 95: 559-570]. Upon completion of the incubations, the specimens were counterstained with hematoxylin. Mouse IgG was used as a negative control. The slides were examined under a light microscope for (a) the extent of integration, as evidenced by the extent of organization of intact and nucleated fat cells, (b) the extent of fibrosis, as evidenced by the amount of collagen and elastic fibrils, (c) the presence of cysts and vacuoles, and (d) the intensity of the inflammatory response, as evidenced by the extent of lymphocyte and macrophage infiltration. Each criterion was graded on a scale of 0 to 5 where 0 = absence, 1 = minimal presence, 2 = minimal to moderate presence, 3 = moderate presence, 4 = moderate to extensive presence, and 5 = extensive presence.

Quantification of macrophage infiltration in the fat grafts was estimated by counting the number of CD68-positive cells in five fields per fat graft in all fat graft sections. Microvascular density (MVD) in fat grafts was determined in five regions of interest where the CD31 antibody signal was the most intense in each section in all of the fat graft sections. The number of macrophages and blood vessels in each region was counted under a light microscope at 400 x magnification. The assessment in each fat graft was made by calculating the mean result in two different sections per fat graft and five different fields of view per section.

### RESULTS

The histological criteria in the PBS-treated fat grafts in the first experiment were not different from those in the second experiment. The extent of integration was higher in the high-dose EPO-treated grafts compared to the low-dose EPO- or PBS-treated grafts, whereas, the extent of cyst formation and fibrosis was lower in the high-dose EPO-treated grafts compared to the low-dose EPO- or PBS-treated grafts (Figures 2A-C). The severity of the inflammatory response as evidenced by CD68-positive cell infiltration in fat grafts in both the low-dose and high-dose EPO-treated fat grafts was lower than that in the PBS-treated fat grafts (Figures 2D-F and 2J). However, the severity of the inflammatory response in the high-dose EPO-treated grafts was significantly lower than that of the low-dose EPO-treated grafts (Figures 2A-C). The extent of integration, cyst formation, and fibrosis in the VEGF-treated grafts was not different from those in the PBS-treated grafts. However, the intensity of the inflammatory response in the VEGF-treated fat grafts was significantly higher than that observed in the PBS-treated fat grafts (Table 3, below).

**Table 3: Histological analysis of the dissected fat grafts**

| | **First Experiment** | | | **Second Experiment** | |
|---|---|---|---|---|---|
| | **PBS** | **Low-Dose EPO** | **High-Dose EPO** | **PBS** | **VEGF** |
| | **(n=10)** | **(n=10)** | **(n=10)** | **(n=10)** | **(n=10)** |
| Integration | 3.3±1.0 | 4.3±0.8 | 4.6±0.7* | 3.6±0.7 | 3.2±0.9 |
| Fibrosis | 2.5±0.9 | 2.1±0.6 | 1.5±0.7* | 2.6±0.5 | 2.9±0.7 |
| Cyst/Vacuoles | 2.8±0.9 | 2.0±0.9 | 1.7±0.7* | 2.9±1.0 | 3.3±1.0 |
| Inflammation | 2.9±1.1 | 1.7±0.5* | 1.3±0.6** | 3.2.0±1.4 | 4.0±1.2* |

| | | | | | |
|---|---|---|---|---|---|
| Of note: Values are presented as mean ± SD n = number of mice EPO = erythropoietin VEGF = vascular endothelial growth factor *P<0.05, **P<0.01 and is the significance of the difference between either the low dose or high dose EPO-treated fat grafts and the PBS-treated grafts. | | | | | |

### EXAMPLE 3

### The effect of EPO on microvascular density in the fat grafts

### MATERIALS AND EXPERIMENTAL PROCEDURES

### Isolation and Preparation of Human Fat Tissue

As described in Example 1, hereinabove.

### Study Design

As described in Example 1, hereinabove.

### Assessment of microvascular density (MVD)

The paraffin-embedded fat graft sections were incubated overnight at room temperature with a monoclonal antibody against tissue CD31 (R&D Systems, Minneapolis MN, USA) as previously described [Li et al. (2005) J Cell Biochem 95:559-570]. Upon completion of the incubation, the specimens were counterstained with hematoxylin. Mouse IgG was used as a negative control. The microvascular density (MVD) was determined in five regions of interest where the CD31 antibody signal was most intense. The number of blood vessels in each region was counted under a light microscope at 40 x magnification.

### RESULTS

As depicted in Figures 2G-I and 2K, the microvascular densities (MVDs) in the two EPO-treated fat grafts were significantly higher than that of the PBS-treated fat graft, and the effect of EPO on MVD was dose-dependent. There were avascular areas, ectatic vessels with edema and perivascular hemorrhage, and a marked reduction in capillary ramification in the PBS-treated fat grafts. In the EPO-treated grafts, there were well-vascularized areas with increased expression of CD31, and numerous endothelial islets (Figures 2G-I and 2K). The extent of MVD was negatively correlated to the extent of macrophage infiltration in the fat grafts (Figure 2L).

### EXAMPLE 4

### The effect of EPO on VEGF content and on the expression levels of angiogenic factors and PKB in the fat grafts

### MATERIALS AND EXPERIMENTAL PROCEDURES

### Isolation and Preparation of Human Fat Tissue

As described in Example 1, hereinabove.

### Study Design

As described in Example 1, hereinabove.

### Western Blotting

The expression levels of the angiogenic factors, bFGF, IGF-1, PDGF-BB VEGFR-2, EPOR and MMP-2, the cell survival factor PKB and phosphorylated PKB, and the pro-apoptotic factors caspase 3 and cytochrome c were determined in homogenates of the harvested fats grafts by Western blotting. Briefly, homogenates of samples of the fat grafts were lysed in RIPA buffer (R&D Systems, MN, USA). A 40 µg aliquot of each lysate was loaded onto SDS-PAGE, and then transferred onto nitrocellulose membranes. Membranes were then incubated with monoclonal antibodies against bFGF, IGF-1, PDGF-BB, MMP-2, PKB, phosphoPKB, caspase 3, and cytochrome c (all purchased from Santa Cruz, CA, USA), or with monoclonal antibodies against VEGFR-2 and EPOR (R&D systems), before a second incubation with a horseradish peroxidase (HRP)-conjugated IgG secondary antibody. An antibody against β-actin (Santa Cruz) was used to normalize protein loading. The resultant bands were quantified by densitometry.

### RESULTS

The VEGF content in the low-dose and high-dose EPO-treated fat grafts was significantly higher compared to the PBS-treated fat grafts. The VEGF content in the high-dose EPO-treated grafts was significantly higher than that in the low-dose EPO-treated graft (Figures 3A-C and 4A). EPO treatment lead to a dose-dependent increase in the expression levels of bFGF, IGF-1, PDGF-BB, MMP-2 PKB and phosphoPKB (Figure 3J). Furthermore, EPO increased both tissue VEGFR-2 and EPOR expression in a dose-dependent manner, as evidenced by immunohistochemical localization of both factors (Figures 3D-I) and by western blot analysis (Figure 4B-C). The VEGF content and the mean expression levels of both VEGFR-2 and EPOR were positively correlated with MVD (Figures 4D-F).

### EXAMPLE 5

### The effect of EPO on the extent of apoptosis in the fat grafts

### MATERIALS AND EXPERIMENTAL PROCEDURES

### Isolation and Preparation of Human Fat Tissue

As described in Example 1, hereinabove.

### Study Design

As described in Example 1, hereinabove.

### Determination of the Extent of Apoptosis in the Fat Grafts

The extent of apoptosis in all fat grafts was assessed by the terminal deoxyuridine triphosphate nick end labeling (TUNEL) assay using a commercial kit (ApopTag® Plus Fluorescein Kit, CHEMICON, CA, USA), in accordance with the manufacturer's instructions. Duplicates were carried out for each sample and were processed by fluorescence-activated cell sorting (FACS, Becton Dickinson, NJ, USA). Data was analyzed using the Macintosh CELLQuest software program (Becton Dickinson).

### RESULTS

The extent of apoptosis in the PBS-treated fat grafts was greater than that observed in the low-dose and high-dose EPO-treated fat grafts (Figure 5A). The extent of apoptosis in the high-dose EPO-treated fat grafts was significantly lower than that observed in the low-dose EPO-treated graft (Figure 5A). Furthermore, EPO lead to a dose-dependent decrease in the expression levels of caspase 3 and cytochrome c (Figure 5B).

### EXAMPLE 6

### The effect of VEGF on microvascular density (MVD) and on the extent of apoptosis in the fat grafts

### MATERIALS AND EXPERIMENTAL PROCEDURES

### Isolation and Preparation of Human Fat Tissue

As described in Example 1, hereinabove.

### Study Design

As described in Example 1, hereinabove.

### Assessment of MVD

As described in Example 3, hereinabove.

### Determination of the Extent of Apoptosis in the Fat Grafts

As described in Example 5, hereinabove.

### RESULTS

The MVD and the extent of apoptosis in the PBS-treated fat grafts in the first experiment were similar to those in the second experiment. The MVD and the VEGF content in the VEGF-treated fat grafts were higher than, but not statistically different from, those in the PBS-treated fat grafts (Figures 6A-B). Furthermore, there was unorganized vessel formation and perivascular hemorrhage in the VEGF-treated fat grafts (data not shown). The extent of apoptosis in the VEGF-treated fat grafts was greater than that in the PBS-treated fat grafts (Figure 6C). There were no statistical differences in the expression levels of caspase 3 and cytochrome c in the PBS-treated and VEGF-treated fat grafts (Figure 6D).

### EXAMPLE 7

### The effect of EPO on endothelial cell tube formation on matrigel

### MATERIALS AND EXPERIMENTAL PROCEDURES

### Isolation and Preparation of Human Fat Tissue

As described in Example 1, hereinabove.

### Study Design

As described in Example 1, hereinabove.

### In vitro Tube Formation of HUVECs on Matrigel

The *in vitro* angiogenic potential of VEGF and EPO was measured by assessment of their ability to form tubes of endothelial cells on matrigel. To this end, human umbilical vein endothelial cells (HUVECs, LONZA, USA) were first cultured on fibronectin-coated 6-well plates in endothelial basal medium-2 (EBM-2, PromoCell, USA) until confluence and then the cells were treated with 0, 20 or 100 IU/ml EPO for 48 hours before their use in the assay (Figure 7A). In a second experiment (Figure 7B), HUVECs were exposed to 0, 100 IU/ml EPO or 200 ng/ml VEGF for 48 hours in EBM-2 with or without 0.25 mg/ml bevacizumab (Avastin®, Genentech, San Francisco, CA, USA), a humanized monoclonal antibody that antagonizes the actions of VEGF. After 48 hours, the untreated HUVECs, the VEGF- and EPO-treated HUVECs, and the VEGF+bevacizumab- and EPO+bevacizumab-treated HUVECs were detached gently by 0.5 % trypsin/EDTA, and then suspended in EBM-2. At the same time, frozen matrigel (Sigma Aldrich, St Louis MO, USA) was thawed, and spread onto 96-well plates (40 µl/well) at room temperature for 30 minutes to allow solidification. The detached untreated HUVECs, VEGF- and EPO-treated HUVECs, and VEGF+bevacizumab- and EPO+bevacizumab-treated HUVECs (5 x 10⁴ cells/150µl EBM-2/well) were placed on the matrigel surface, and then incubated at 37 °C for 24 hours in EBM-2. After plating on the matrigel, the VEGF- and EPO-treated HUVECs and VEGF+bevacizumab- and EPO+bevacizumab-treated HUVECs were treated again with identical concentrations of EPO, VEGF, and bevacizumab, respectively. After 24 hours, the non-integrated cells were removed by washing and tube formation on the matrigel was assessed under a light microscope at 10 x magnification. The tubular structures were graded semiquantitatively by evaluating the presence and stages of tube formation on a scale of 0 to 5 as follows: 0 = well separated individual cells, 1 = cells had begun to migrate and align themselves, 2 = visible capillary tubes and no sprouting, 3 = visible sprouting of new capillary tubes, 4 = early formation of closed polygons, 5 = development of complex mesh-like structures. Four random high-power fields in each sample were examined. The results from each examiner were then pooled in order to calculate the mean value for each criterion for each sample in each group.

### HUVEC proliferation

To investigate EPO-induced angiogenesis through mechanisms involving pro-angiogenic factors, the present inventor measured the proliferation of EPO-treated HUVECs in the presence of various pro-angiogenic factor inhibitors. To this end, HUVECs (2 x 10⁵ cells/well) were cultured on fibronectin-coated 12-well plates in EBM-2. The cultured HUVECs were treated with or without 100 IU/ml EPO for 48 hours, and then exposed for 3 hours to (a) 0.25 mg/ml bevacizumab, (b) 100 nM of PD173074; an inhibitor of bFGF (Calbiochem, San Diego, CA), (c) 20 µM of tyrphostin AG 1296; a selective inhibitor of PDGF (Sigma), (d) a combination of bevacizumab, PD173074 and tyrphostin, and to (e) 100 nM wortmannin; a phosphatidylinositol 3-kinaz (PI 3-K) inhibitor (Sigma). Upon the completion of the experiment, the cells were washed with PBS and then incubated with 1 µCi/ml medium [³H]-thymidine (NEN, Boston, MA, USA) for 5 h at 37 °C. Thereafter, 0.5 ml cold 10 % Trichloroacetic acid (TCA) was added into each well for another 30 min at 4 °C. To extract the ³H-thymidine labeled DNA, 0.5 ml 1N NaOH was added to each well for 10 min at room temperature, and then 0.5 ml IN HCl was added and mixed well. Samples of mixture solution (0.5 ml) was taken from each well and added to scintillation vials for the measurement of [³H]-thymidine incorporation to DNA (cpm/mg protein). Duplicate cell counts were averaged for 3 experiments. Data were expressed as the percentage of control.

### RESULTS

As described in Figure 7A, EPO enhanced human umbilical vein endothelial cell (HUVEC) tube formation in a dose-dependent manner. Furthermore, both VEGF and EPO significantly enhanced HUVEC tube formation (Figure 7B). Tube formation was substantially reduced in VEGF + bevacizumab-treated HUVECs, but not in the EPO + bevacizumab-treated HUVECs (Figures 7B-H).

The VEGF inhibitor, bFGF inhibitor and PDGF inhibitor each reduced HUVEC proliferation significantly, whereas either a combination of the 3 inhibitors together or wortmannin alone abolished HUVEC proliferation (Figure 7 I). EPO normalized HUVEC proliferation in the presence of any of the inhibitors, but had no effect on HUVEC proliferation in the presence of a combination of the 3 inhibitors together or in the presence of wortmannin alone (Figure 7 I). Although the invention has been described in conjunction with specific embodiments thereof, it is evident that many alternatives, modifications and variations falling under the scope of the claims will be apparent to those skilled in the art. Citation or identification of any reference in this application shall not be construed as an admission that such reference is available as prior art to the present invention. To the extent that section headings are used, they should not be construed as necessarily limiting.

## Claims

1. Erythropoietin for use in enhancing implanted fat cell survival for reconstruction surgery in a subject in need thereof, wherein a dose of Erythropoietin for local administration ranges between 1-1000 IU per injection per 1,000,000 fat cells or wherein the dose of Erythropoietin for systemic administration ranges between 10 - 7500 IU per kg body weight.

2. The Erythropoietin for use according to claim 1, wherein said reconstruction surgery comprises a mastectomy or lumpectomy.

3. The Erythropoietin for use according to claim 1, wherein said fat cell is used to fill a skin depression.

4. The Erythropoietin for use according to claim 1, wherein said fat cell is further treated with Erythropoietin prior to implantation thereof.

5. The Erythropoietin for use according to claim 1, wherein said Erythropoietin is directly injected into said fat cells.

6. The Erythropoietin for use according to claim 1, further comprising administering at least one factor selected from the group consisting of an extracellular matrix component, a growth factor, a hormone, an angiogenic factor, a coagulation factor, a cytokine, a chemokine, an enzyme, a neurotransmitter, a vitamin, a carbohydrate, an ion, an iron chelator, a fatty acid, an antibiotic and an amino acid.

7. The Erythropoietin for use according to claim 1, wherein said fat cell comprises a non-autologous cell.

8. The Erythropoietin for use according to claim 7, wherein said non-autologous cell is an allogeneic cell or a xenogeneic cell.

9. The Erythropoietin for use according to claim 1, wherein said fat cell comprises an autologous cell.

10. A pharmaceutical composition comprising a population of fat cells and Erythropoietin at a dose of about 1-1000 IU per injection per 1,000,000 fat cells.

11. The pharmaceutical composition of claim 10, further comprising at least one factor selected from the group consisting of an extracellular matrix component, a growth factor, a hormone, an angiogenic factor, a coagulation factor, a cytokine, a chemokine, an enzyme, a neurotransmitter, a vitamin, a carbohydrate, an ion, an iron chelator, a fatty acid, an antibiotic, and an amino acid.

12. Use of Erythropoietin in enhancing implanted fat cell survival for treating wrinkles in a subject in need thereof, wherein a dose of Erythropoietin for local administration ranges between 1-1000 IU per injection per 1,000,000 fat cells or wherein the dose of Erythropoietin for systemic administration ranges between 10 - 7500 IU per kg body weight, wherein the use is non-therapeutic.

13. The use according to claim 12, wherein said fat cell is further treated with Erythropoietin prior to implantation thereof.

14. The use according to claim 12, wherein said Erythropoietin is directly injected into said fat cells.

15. The use according to claim 12, further comprising administering at least one factor selected from the group consisting of an extracellular matrix component, a growth factor, a hormone, an angiogenic factor, a coagulation factor, a cytokine, a chemokine, an enzyme, a neurotransmitter, a vitamin, a carbohydrate, an ion, an iron chelator, a fatty acid, an antibiotic and an amino acid.

16. The use according to claim 12, wherein said fat cell comprises a non-autologous cell.

17. The use according to claim 12, wherein said fat cell comprises an autologous cell.

## Patentansprüche

1. Erythropoietin zur Anwendung bei der Förderung des Überlebens implantierter Fettzellen für die Rekonstruktionschirurgie bei Betroffenen, wobei eine Dosis Erythropoietin zur lokalen Applikation zwischen 1 und 1.000 IU je Injektion pro 1.000.000 Fettzellen aufweist oder wobei die Dosis Erythropoietin zur systemischen Applikation zwischen 10 und 7.500 IU je Kilogramm Körpergewicht aufweist.

2. Erythropoetin zur Anwendung nach Anspruch 1, wobei die besagte Rekonstruktionschirurgie eine Mastektomie oder Lumpektomie beinhaltet.

3. Erythropoietin zur Anwendung nach Anspruch 1, wobei die besagte Fettzelle zum Füllen einer Hautvertiefung eingesetzt wird.

4. Erythropoietin zur Anwendung nach Anspruch 1, wobei die besagte Fettzelle vor ihrer Implantation mit Erythropoietin weiter behandelt wird.

5. Erythropoietin zur Anwendung nach Anspruch 1, wobei das besagte Erythropoetin direkt in die besagten Fettzellen injiziert wird.

6. Erythropoietin zur Anwendung nach Anspruch 1, weiterhin aufweisend die Gabe mindestens eines Faktors, der ausgewählt ist aus der Gruppe bestehend aus einer extrazellulären Matrixkomponente, einem Wachstumsfaktor, einem Hormon, einem angiogenen Faktor, einem Koagulationsfaktor, einem Cytokin, einem Chemokin, einem Enzym, einem Neurotransmitter, einem Vitamin, einem Kohlenhydrat, einem Ion, einem Eisenchelator, einer Fettsäure, einem Antibiotikum und einer Aminosäure.

7. Erythropoietin zur Anwendung nach Anspruch 1, wobei die besagte Fettzelle eine nicht-autologe Zelle aufweist.

8. Erythropoietin zur Anwendung nach Anspruch 7, wobei die besagte nicht-autologe Zelle eine allogene Zelle oder eine xenogene Zelle ist.

9. Erythropoietin zur Anwendung nach Anspruch 1, wobei die besagte Fettzelle eine autologe Zelle aufweist.

10. Eine pharmazeutische Zubereitung, die eine Population von Fettzellen und Erythropoietin mit einer Dosis von etwa 1 bis 1.000 IU je Injektion pro 1.000.000 Fettzellen aufweist.

11. Die pharmazeutische Zubereitung nach Anspruch 10, weiterhin aufweisend mindestens einen Faktor, der ausgewählt ist aus einer Gruppe bestehend aus einer extrazellulären Matrixkomponente, einem Wachstumsfaktor, einem Hormon, einem angiogenen Faktor, einem Koagulationsfaktor, einem Cytokin, einem Chemokin, einem Enzym, einem Neurotransmitter, einem Vitamin, einem Kohlenhydrat, einem Ion, einem Eisenchelator, einer Fettsäure, einem Antibiotikum und einer Aminosäure.

12. Einsatz von Erythropoietin zur Förderung des Überlebens implantierter Fettzellen für die Behandlung von Falten bei Betroffenen, wobei eine Dosis Erythropoietin zur lokalen Applikation von 1 bis 1.000 IU je Injektion pro 1.000.000 Fettzellen aufweist oder wobei die Dosis Erythropoietin zur systemischen Applikation zwischen 10 und 7.500 IU je Kilogramm Körpergewicht aufweist, wobei die Anwendung nicht therapeutisch ist.

13. Einsatz nach Anspruch 12, wobei die besagte Fettzelle vor ihrer Implantation ferner mit Erythropoietin behandelt wird.

14. Einsatz nach Anspruch 12, wobei das besagte Erythropoietin direkt in besagte Fettzellen injiziert wird.

15. Einsatz nach Anspruch 12, weiterhin aufweisend die Gabe mindestens eines Faktors, der ausgewählt ist aus der Gruppe bestehend aus einer extrazellulären Matrixkomponente, einem Wachstumsfaktor, einem Hormon, einem angiogenen Faktor, einem Koagulationsfaktor, einem Cytokin, einem Chemokin, einem Enzym, einem Neurotransmitter, einem Vitamin, einem Kohlenhydrat, einem Ion, einem Eisenchelator, einer Fettsäure, einem Antibiotikum und einer Aminosäure..

16. Einsatz nach Anspruch 12, wobei die besagte Fettzelle eine nicht-autologe Zelle aufweist.

17. Einsatz nach Anspruch 12, wobei die besagte Fettzelle eine autologe Zelle aufweist.

## Revendications

1. Érythropoïétine pour une utilisation dans l'amélioration de la survie de cellules adipeuses implantées pour une chirurgie reconstructive chez un sujet en ayant besoin, dans laquelle une dose d'érythropoïétine pour une administration locale va de 1 à 1 000 IU par injection pour 1 000 000 cellules adipeuses ou dans laquelle la dose d'érythropoïétine pour une administration systémique va de 10 à 7 500 IU par kg de masse corporelle.

2. Érythropoïétine pour une utilisation selon la revendication 1, dans laquelle ladite chirurgie reconstructive comprend une mastectomie ou une lumpectomie.

3. Érythropoïétine pour une utilisation selon la revendication 1, dans laquelle ladite cellule adipeuse est utilisée pour remplir une dépression cutanée.

4. Érythropoïétine pour une utilisation selon la revendication 1, dans laquelle ladite cellule adipeuse est en outre traitée avec une érythropoïétine avant l'implantation de celle-ci.

5. Érythropoïétine pour une utilisation selon la revendication 1, dans laquelle ladite érythropoïétine est directement injectée dans lesdites cellules adipeuses.

6. Érythropoïétine pour une utilisation selon la revendication 1, comprenant en outre l'administration d'au moins un facteur sélectionné parmi le groupe constitué d'un composant matriciel extracellulaire, d'un facteur de croissance, d'une hormone, d'un facteur angiogénique, d'un facteur de coagulation, d'une cytokine, d'une chimiokine, d'une enzyme, d'un neurotransmetteur, d'une vitamine, d'un glucide, d'un ion, d'un chélateur d'ions, d'un acide gras, d'un antibiotique et d'un acide aminé.

7. Érythropoïétine pour une utilisation selon la revendication 1, dans laquelle ladite cellule adipeuse comprend une cellule non autologue.

8. Érythropoïétine pour une utilisation selon la revendication 7, dans laquelle ladite cellule non autologue est une cellule allogénique ou une cellule xénogénique.

9. Érythropoïétine pour une utilisation selon la revendication 1, dans laquelle ladite cellule adipeuse comprend une cellule autologue.

10. Composition pharmaceutique comprenant une population de cellules adipeuses et une érythropoïétine à raison d'une dose d'environ 1 à 1 000 IU par injection pour 1 000 000 cellules adipeuses.

11. Composition pharmaceutique selon la revendication 10, comprenant en outre au moins un facteur sélectionné parmi le groupe constitué d'un composant matriciel extracellulaire, d'un facteur de croissance, d'une hormone, d'un facteur angiogénique, d'un facteur de coagulation, d'une cytokine, d'une chimiokine, d'une enzyme, d'un neurotransmetteur, d'une vitamine, d'un glucide, d'un ion, d'un chélateur d'ions, d'un acide gras, d'un antibiotique, et d'un acide aminé.

12. Utilisation de l'érythropoïétine dans l'amélioration de la survie de cellules adipeuses implantées pour le traitement de rides chez un sujet en ayant besoin, dans laquelle une dose d'érythropoïétine pour une administration locale va de 1 à 1 000 IU par injection pour 1 000 000 cellules adipeuses ou dans laquelle la dose d'érythropoïétine pour une administration systémique va de 10 à 7 500 IU par kg de masse corporelle, dans laquelle l'utilisation est non-thérapeutique.

13. Utilisation selon la revendication 12, dans laquelle ladite cellule adipeuse est en outre traitée avec une érythropoiétine avant l'implantation de celle-ci.

14. Utilisation selon la revendication 12, dans laquelle ladite érythropoiétine est directement injectée dans lesdites cellules adipeuses.

15. Utilisation selon la revendication 12, comprenant en outre l'administration d'au moins un facteur sélectionné parmi le groupe constitué d'un composant matriciel extracellulaire, d'un facteur de croissance, d'une hormone, d'un facteur angiogénique, d'un facteur de coagulation, d'une cytokine, d'une chimiokine, d'une enzyme, d'un neurotransmetteur, d'une vitamine, d'un glucide, d'un ion, d'un chélateur d'ions, d'un acide gras, d'un antibiotique et d'un acide aminé.

16. Utilisation selon la revendication 12, dans laquelle ladite cellule adipeuse comprend une cellule non autologue.

17. Utilisation selon la revendication 12, dans laquelle ladite cellule adipeuse comprend une cellule autologue.
